# EUROPÄISCHE PATENTSCHRIFT

(11) **EP 2 760 592 B1**
(45) Veröffentlichungstag und Bekanntmachung des Hinweises auf die Patenterteilung: **21.10.2015**
(21) Anmeldenummer: 11810595.6
(22) Anmeldetag: 22.12.2011
(51) Int. Cl.: B05B 11/00

(54) **DOSIERSPENDER**
METERING DISPENSER
DISTRIBUTEUR DOSEUR

(30) Priorität: 30.09.2011 DE 102011114568; 18.10.2011 DE 102011116054
(43) Veröffentlichungstag der Anmeldung: 06.08.2014
(73) Patentinhaber: AVENIDA GmbH & Co. KG, 35037 Marburg (DE)
(72) Erfinder: RITZENHOFF, Andreas Franz Christian, 35039 Marburg (DE); BODENBENDER, Manfred, 35274 Kirchhain-Großseelheim (DE); STOCK, Jörg, 35287 Amöneburg (DE); ARNOLD, Oliver, 35232 Dautphetal- Allendorf (DE); NEUNER, Charles, Amityville, New York 11701 (US)
(74) Vertreter: advotec.
(86) Internationale Anmeldenummer: PCT/EP2011/073811
(87) Internationale Veröffentlichungsnummer: WO 2013/044998

(56) Entgegenhaltungen:
- DE-U1-202009 014 316

## Beschreibung

Die Erfindung betrifft einen Dosierspender zur dosierten Abgabe von zumindest zwei in jeweils einem Reservoir aufgenommenen Komponenten, wobei jedem Reservoir eine Fördereinrichtung zugeordnet ist und die Fördereinrichtungen mit einer Betätigungseinrichtung zur Ausgabe der Komponenten aus einer mit den Reservoirs verbundenen Ausgabeeinrichtung versehen sind, wobei die Betätigungseinrichtung mittelbar über eine Dosiereinrichtung auf die Fördereinrichtungen wirkt und die Dosiereinrichtung mit einer Stelleinrichtung versehen ist, mit deren Betätigung die Stellung eines auf die Fördereinrichtungen wirkenden Übertragungselements zur Einstellung des Mengenverhältnisses der Komponenten veränderbar ist.

Aus der EP 1 104 336 A1 ist ein Dosierspender der eingangs genannten Art bekannt, der eine Stelleinrichtung zur Einstellung des Mengenverhältnisses der Komponenten aufweist, die ein um eine Schwenkachse verschwenkbares Übertragungselement aufweist, das bei Ausführung einer Schwenkbewegung auf die Fördereinrichtungen wirkt. Zur Einstellung der Dosiereinrichtung wird mittels einer Stelleinrichtung die Schwenkachse des Übertragungselements um die Hochachse des Dosierspenders gedreht. Nach Einstellung der Dosiereinrichtung wird zur Ausgabe der Komponenten aus den Reservoirs auf einen Betätigungsabschnitt des Übertragungselements eine Betätigungskraft ausgeübt, derart, dass infolge der Einwirkung der Betätigungskraft das Übertragungselement um die quer zur Längsachse des Dosierspenders verlaufende Schwenkachse verschwenkt wird.

Aus der DE 20 2009 014 316 U1 ist ebenfalls ein Dosierspender der eingangs genannten Art bekannt.

Die bei dem bekannten Dosierspender notwendige translatorische Betätigung zur Ausführung einer Schwenkbewegung des Übertragungselements um die quer zur Längsachse orientierte Schwenkachse des Übertragungselements erlaubt in der Praxis eine nur sehr ungenaue Dosierung der aus der Ausgabeeinrichtung ausgegebenen Gesamtmenge der beiden Komponenten. Die Ausgabe einer reproduzierbaren Ausgabemenge der beiden Komponenten ist mit der bekannten Betätigungseinrichtung nur möglich bei Ausführung eines vollständigen Ausschlags des Übertragungselements um die Schwenkachse. Dem hingegen ist die Ausgabe lediglich einer Teilmenge der möglichen Maximalmenge kaum reproduzierbar möglich, da die reproduzierbare Ausgabe einer Teilmenge eine entsprechend reproduzierbare Ausführung eines Teilausschlags des Übertragungselementes voraussetzt. Die Ausführung eines definierten Ausschlags des Betätigungselements ist jedoch bei dem insgesamt nur sehr geringen Weg, den das Übertragungselement bei einer Schwenkbewegung durchführt, kaum möglich.

Der vorliegenden Erfindung liegt daher die Aufgabe zugrunde, einen Dosierspender vorzuschlagen, der eine genaue Dosierung einer aus zwei Komponenten zusammengesetzten Ausgabemenge ermöglicht.

Darüber hinaus ist es eine Aufgabe der Erfindung, einen Dosierspender vorzuschlagen, der einfacher und somit für den Benutzer angenehmer zu betätigen ist.

Zur Lösung dieser Ausgabe weist der erfindungsgemäße Dosierspender die Merkmale des Anspruchs 1 auf.

Bei dem erfindungsgemäßen Dosierspender sind sowohl die Stelleinrichtungen zur Einstellung des Mengenverhältnisses der Komponenten als auch die Betätigungseinrichtung zur Ausgabe der Komponenten aus der Ausgabeeinrichtung zu ihrer Betätigung um eine Längsachse des Dosierspenders drehbar.

Bei der Erfindung wird von der Erkenntnis Gebrauch gemacht, dass es grundsätzlich von dem Benutzer eines Dosierspenders als angenehmer empfunden wird, die Betätigungen oder Einstellungen, die von ihm an dem Dosierspender beim Gebrauch vorzunehmen sind, mittels gleichartiger händischer Bewegungen auszuführen. Dies insbesondere deswegen, da bei dem erfindungsgemäßen Dosierspender zwischen der Einstellung des Mischungsverhältnisses der Komponenten und der Betätigung zur Ausgabe der Komponenten in dem eingestellten Mischungsverhältnis keine Änderung in der Handhabung des Dosierspenders vorzunehmen ist. Vielmehr ist in beiden Fällen jeweils eine Drehbewegung um die Längsachse des Dosierspenders auszuführen.

Darüber hinaus liegt der Erfindung die Erkenntnis zugrunde, dass es aus ergonomischen Gründen für den Benutzer eines Dosierspenders wesentlich einfacher ist, eine reproduzierbare Drehbewegung auszuführen, als dies bei einer translatorischen Bewegung der Fall ist, insbesondere dann, wenn der für die Auflösung der translatorischen Bewegung zur Verfügung stehende Stellweg eher kurz ist.

Bei einer bevorzugten Ausführungsform des Dosierspenders weist die Stelleinrichtung der Dosiereinrichtung ein um die Längsachse eines Reservoirgehäuses, in dem die Reservoirs zur Aufnahme der Komponenten und die Fördereinrichtungen aufgenommen sind, ein drehbares Stellgehäuse auf, in dem das Übertragungselement gegenüber dem Stellgehäuse drehstarr und auf der Längsachse des Stellgehäuses zur Betätigung der Fördereinrichtungen axial verschiebbar angeordnet ist, wobei das Übertragungselement eine ringförmig oder als Ringsegment ausgebildete Kontaktoberfläche aufweist, mit einer sich umlaufend ändernden Oberflächenkontur, die mit den Fördereinrichtungen zusammenwirkt.

Bei dieser vorteilhaften Ausführungsform ermöglicht demnach die besondere Ausgestaltung der Stelleinrichtung eine Umwandlung einer durch Einwirkung auf das Stellgehäuse von außen durch den Benutzer aufgebrachten manuellen Drehbewegung des Stellgehäuses in eine axiale oder translatorische Bewegung des Übertragungselements, sodass einerseits eine ergonomisch günstige Drehverstellung der Stelleinrichtung durch den Benutzer möglich ist, andererseits durch die axiale Stellbewegung des Übertragungselements eine funktionssichere Betätigung der Fördereinrichtungen möglich ist, bei der stets die horizontale Ausrichtung des Übertragungselements bezogen auf eine vertikale Ausrichtung der Längsachse des Dispensers erhalten bleibt.

Insbesondere ergeben sich bei einer Beaufschlagung der Fördereinrichtungen durch das Übertragungselement keine Relativbewegungen zwischen dem Übertragungselement und den Fördereinrichtungen, die zu Abnutzungserscheinungen führen könnten, welche dem zuverlässigen und in gewünschter Weise lange währenden und sicheren Gebrauch des Dispensers entgegen stehen könnten.

Die Einstellung des gewünschten Mengenverhältnisses zwischen den beiden für die Ausgabe vorgesehenen Komponenten erfolgt lediglich durch eine Drehung des Übertragungselements um die Hochachse des Dosierspenders bzw. des Stellgehäuses so weit, bis die Relativstellung des Übertragungselements gegenüber den Fördereinrichtungen erreicht ist, in der die das Mengenverhältnis definierenden unterschiedlichen Kontaktstellen des Übertragungselements den Fördereinrichtungen zugeordnet sind.

Vorteilhafterweise kann die Betätigungseinrichtung zur Ausgabe der Komponenten ein um die Längsachse des Dosierspenders relativ zum Stellgehäuse drehbares Betätigungsgehäuse aufweisen, das mit einer Führungsanordnung zur axialen Verschiebbarkeit des Übertragungselementes versehen ist, wobei eine erste im Betätigungsgehäuse ausgebildeten Führungseinrichtung der Führungsanordnung zur Umwandlung einer Drehbewegung des Betätigungsgehäuses in eine axiale Bewegung des Übertragungselements mit einer zweiten unabhängig vom Betätigungsgehäuse ausgebildeten Führungseinrichtung der Führungsanordnung zusammenwirkt.

Eine besonders betriebssichere und zugleich einfach ausführbare Ausgestaltung der zweiten Führungseinrichtung wird möglich, wenn die zweite Führungseinrichtung ein Führungsteil mit einer axial ausgerichteten Führungskulisse und eine am Übertragungselement positionierte Zapfenanordnung mit zumindest einem radialen Führungszapfen aufweist, der die Führungskulisse durchdringt und mit seinem Kontaktende mit der ersten Führungseinrichtung des Betätigungsgehäuses zusammenwirkt.

Besonders vorteilhaft ist es auch, wenn das Betätigungsgehäuse zur Ausbildung der ersten Führungseinrichtung auf seiner koaxial zum Führungsteil des Stellgehäuses angeordneten Innenwandung eine Führungsbahn mit einer mit dem Kontaktende des Führungszapfens zusammenwirkenden Kontaktkontur aufweist, die die axiale Bewegung des Übertragungselements steuert, sodass aufgrund der koaxialen Anordnung des Betätigungsgehäuses zum Stellgehäuse eine insgesamt kompakte Ausgestaltung des Dispensers möglich wird.

Eine Drehrichtungssperre oder Definition einer möglich Drehrichtung kann erreicht werden, wenn die im Führungsteil ausgebildete, axial ausgerichtete Führungskulisse an ihrem unteren Ende zur Definition einer Drehrichtung der Betätigungseinrichtung eine Zapfenfalle zur Aufnahme des radialen Führungszapfens aufweist.

Eine besonders vorteilhafte Ausgestaltung des Dosierspenders, die zu einer weiteren Erhöhung der Reproduzierbarkeit definierter Ausgabemengen des Dispensers beiträgt, weist zur Definition einer Ausgangsstellung der Betätigungseinrichtung, in der die Fördereinrichtungen nicht durch das Übertragungselement beaufschlagt werden, zwischen einem am Führungsteil ausgebildeten Anschlag und dem Übertragungselement eine Federeinrichtung auf, sodass die Ausgangsstellung für den Benutzer dadurch haptisch genau erfassbar ist, dass in der Ausgangsstellung ein Widerstand gegen die Drehverstellung der Betätigungseinrichtung minimiert ist.

Besonders vorteilhaft ist es, wenn der Anschlag am axialen Ende eines am Boden des Führungsteils angeordneten Zapfens ausgebildet ist, der sich durch eine in einem Boden einer zentralen hülsenförmigen Vertiefung des Übertragungselements ausgebildete Öffnung erstreckt, und wenn die Federeinrichtung als eine Schraubenfeder ausgebildet ist, die in einem zwischen dem Zapfen und der Vertiefung ausgebildeten Ringraum angeordnet ist und sich zwischen dem Anschlag und dem Boden erstreckt. Hierdurch ist eine sichere Gehäusung für die Federeinrichtung geschaffen, sodass die Funktion der Federeinrichtung auch bei einem lange währenden Gebrauch des Dosierspenders sichergestellt ist.

Alternativ zur Ausgestaltung des Dosierspenders mit einer Federeinrichtung besteht auch die vorteilhafte Möglichkeit, zur Definition einer Ausgangsstellung der Betätigungseinrichtung, an einem axialen Ende eines im Führungsteil angeordneten Rastzapfens eine Rasteinrichtung vorzusehen, die bei dem in Ausgangsstellung befindlichen Übertragungselement einen Rand einer zentralen Öffnung im Übertragungselement hintergreift.

Bei dieser Ausführungsform des Dosierspenders, bei der die konstruktiven Maßnahmen zur Definition der Ausgangsstellung der Betätigungseinrichtung in besonders einfacher Art und Weise ausgeführt sind, erweist es sich zur betriebssicheren Funktion als vorteilhaft, wenn die in der Innenwandung des Betätigungsgehäuses angeordnete Führungsbahn als Führungsnut ausgebildet ist, deren parallel zueinander verlaufende Nutränder den Führungszapfen zwischen sich aufnehmen.

Besonders vorteilhaft ist es auch, wenn zur Ausbildung der zweiten Führungseinrichtung das Führungsteil durch das Stellgehäuse ausgebildet ist, und die Zapfenanordnung mit dem zumindest einen Führungszapfen am Übertragungselement ausgebildet ist, derart, dass bei einer Drehung des Stellgehäuses eine entsprechende Drehung des Führungsteils erfolgt. Hierdurch kommt dem Stellgehäuse eine vorteilhafte Doppelfunktion zu, so dass eine entsprechende Reduktion der Menge an Einzelteile möglich wird.

Bei einer besonders vorteilhaften Ausführungsform des Dosierspenders ist zur definierten Relativanordnung des Stellgehäuses zum Reservoirgehäuse eine Rasteinrichtung zwischen dem Stellgehäuse und dem Reservoirgehäuse ausgebildet, die eine Mehrzahl von Raststellungen für eine definierte Zuordnung von auf der Kontaktoberfläche ausgebildeten Kontaktstellen des Übertragungselements und auf Förderkolben der Fördereinrichtungen wirkenden Stößeln aufweist. Zum einen erleichtert die Rasteinrichtung das wiederholte Auffinden einer relativen Drehstellung zwischen dem Stellgehäuse und dem Reservoirgehäuse zur reproduzierbaren Einstellung eines Mischungsverhältnisses. Zum anderen kann die Einstellung eines einmal eingestellten Mischungsverhältnisses zwischen den Komponenten durch die Rasteinrichtung gesichert werden.

Wenn die Rasteinrichtung als eine modulare, zwischen das Reservoirgehäuse und das Stellgehäuse einsetzbare Rasteinheit ausgebildet ist, die drehrichtungsabhängig zur Relativdrehung des Stellgehäuses gegenüber dem Reservoirgehäuse betätigbar ist, kann durch einen einfachen Austausch der Rasteinrichtung die Drehrichtung eingestellt werden.

Besonders vorteilhaft ist es, wenn die Rasteinheit ein erstes, drehfest mit dem Reservoirgehäuse verbindbares, ringförmiges Rastelement und ein zweites, drehfest mit dem Stellgehäuse verbindbares, ringförmiges Rastelement aufweist, wobei die Rastelemente über einen in einer gemeinsamen Ringebene ausgebildeten, durch Rastvorsprünge hergestellten Rasteingriff zusammenwirken, und die Rastvorsprünge durch eine Sperrklinken-Verzahnung ausgebildet sind, so die Rasteinheit mit geringem Materialeinsatz und sehr platzsparend ausführbar ist.

Ein elastisch nachgiebiger Rasteingriff wird möglich, wenn eines der beiden Rastelemente die Sperrklinken-Verzahnung lediglich in einem Ringsegment aufweist.

Eine besonders exakte Relativausrichtung zwischen dem Reservoirgehäuse und dem Stellgehäuse wird möglich, wenn die Rasteinrichtung eine mit dem Reservoirgehäuse verbundene Rastachse aufweist, die zur Ausbildung eines Rasteingriffs in eine als Rasthülse ausgebildete Nabe des Übertragungselements eingreift.

Zur Ausbildung des Rasteingriffs ist es vorteilhaft, wenn auf dem Umfang der Rastachse ausgebildete Rastvorsprünge mit auf der Bohrungswandung der Hülse ausgebildeten Rastvorsprüngen zusammenwirken.

Eine besonders betriebssicher Ausgestaltung wird möglich, wenn die Rastvorsprünge durch eine Sperrklinken-Verzahnung ausgebildet sind.

Bei einer vorteilhaften Vatiante zur Ausbildung der zweiten Führungseinrichtung ist das Führungsteil drehfest mit dem Reservoirgehäuse verbunden, und die Zapfenanordnung ist als Führungsring mit zumindest einem Führungszapfen ausgebildet ist. Darüber hinaus ist das Übertragungselement im Führungsring drehbar aufgenommen und mittels einer radialen Eingriffseinrichtung drehfest mit dem Stellgehäuse verbunden, derart, dass bei einer Drehung des Stellgehäuses dieses eine Relativdrehung gegenüber dem Führungsgehäuse ausführt. Auf diese Art und Weise kann verhindert werden, dass bei einer Beaufschlagung der Stößel der Fördereinrichtungen die Stößel durch ein Drehmoment beansprucht werden.

Wenn die Eingriffseinrichtung eine Stellhülse aufweist, in der das Übertragungselement drehfest und axial verschiebbar aufgenommen ist, wobei die Stellhülse über einen Stellzapfen, der das Führungsteil in einer Stellnut radial durchdringt, eine Eingriffsverbindung mit dem Stellgehäuse ausbildet, kann das Stellgehäuse als Stellring ausgebildet werden.

Wenn zur definierten Relativanordnung des Stellgehäuses eine Rasteinrichtung zwischen der Stellhülse und dem Führungsteil ausgebildet ist, die eine Mehrzahl von Raststellungen für eine definierte Zuordnung von auf der Kontaktoberfläche ausgebildeten Kontaktstellen des Übertragungselements und auf Förderkolben der Fördereinrichtungen wirkenden Stößeln aufweist, kann trotz einer möglichen einfachen Ausgestaltung des Stellgehäuses als Ring eine reproduzierbare Einstellung ermöglicht werden.

Wenn gemäß einer besonders vorteilhaften Ausführungsform die Kontaktstellen auf der Kontaktoberfläche des Übertragungselements durch in einer Stufenfolge angeordnete Kontaktabsätze gebildet sind, ist sichergestellt, dass trotz einer sich über den Umfang des Übertragungselements ändernden Oberflächenkontur bzw. Topografie unabhängig von der Relativstellung des Übertragungselements zu den Stößeln der Fördereinrichtungen stets übereinstimmende Kontaktverhältnisse zwischen dem Übertragungselement und dem Stößel ausgebildet sind.

Wenn darüber hinaus die Kontaktabsätze durch Sackbohrungen ausgebildet sind, die in einer horizontalen Oberfläche des Übertragungselements angeordnet sind, ist eine besonders formgenaue Anpassung zwischen den Kontaktstellen und den Stößeln der Fördereinrichtungen möglich, womit einem ansonsten möglichen Verschleiß entgegengewirkt wird. Vorteilhafte Ausführungsformen des Dosierspenders werden nachfolgend anhand der Zeichnungen näher erläutert.

Es zeigen:
- **Fig. 1:**: Eine Ausführungsform eines Dosierspenders mit einem Reservoirgehäuse mit Handhabungseinrichtung;
- **Fig. 2:**: den in **Fig. 1** dargestellten Dosierspender in einer Schnittdarstellung gemäß Schnittlinienverlauf II-II in **Fig. 1****;**
- **Fig. 3:**: eine Schnittdarstellung gemäß Schnittlinienverlauf III-III in **Fig. 2****;**
- **Fig. 4:**: die aus einer Stelleinrichtung und einer Betätigungseinrichtung gebildete Handhabungseinrichtung des in **Fig. 1** dargestellten Dosierspenders;
- **Fig. 5:**: eine Explosionsdarstellung der in **Fig. 4** dargestellten Handhabungseinrichtung;
- **Fig. 6:**: ein Übertragungselement der in **Fig. 5** dargestellten Handhabungseinrichtung in Draufsicht;
- **Fig. 7:**: eine isometrische Darstellung eines in **Fig. 5** dargestellten Stellgehäuses;
- **Fig. 8:**: eine Schnittdarstellung des in **Fig. 6** dargestellten Übertragungselements gemäß Schnittlinienverlauf VIII-VIII in **Fig. 6****;**
- **Fig. 9:**: eine Draufsicht auf das in das Stellgehäuse eingesetzte Übertragungselement;
- **Fig. 10:**: eine Schnittdarstellung der in **Fig. 4** dargestellten Handhabungseinrichtung mit einem in Ausgangsstellung angeordneten Übertragungselement;
- **Fig. 11:**: eine Schnittdarstellung der in **Fig. 10** dargestellten Handhabungseinrichtung gemäß Schnittlinienverlauf XI-XI in **Fig. 10****;**
- **Fig. 12:**: eine Schnittdarstellung der in **Fig. 10** dargestellten Handhabungseinrichtung gemäß Schnittlinienverlauf XII-XII in **Fig. 10****;**
- **Fig. 13:**: eine Einzeldarstellung eines gemäß der Darstellung in **Fig. 11** mit einem Führungsteil des Stellgehäuses koaxial verbundenen Betätigungsgehäuses;
- **Fig. 14:**: eine weitere Ausführungsform der Handhabungseinrichtung in einer **Fig. 12** entsprechenden Darstellung;
- **Fig. 15:**: ein Betätigungsgehäuse zur Kombination mit dem in **Fig. 14** dargestellten Stellgehäuse der Handhabungseinrichtung;
- **Fig. 16:**: eine weitere Ausführungsform eines Dosierspenders in Längsschnittdarstellung;
- **Fig. 17**:: eine Explosionsdarstellung der Handhabungseinrichtung des in **Fig. 16** dargestellten Dosierspenders;
- **Fig. 18:**: eine weitere Ausführungsform eines Dosierspenders in Längsschnittdarstellung;
- **Fig. 19:**: eine Explosionsdarstellung der Handhabungseinrichtung des in **Fig. 18** dargestellten Dosierspenders;
- **Fig. 20:**: eine Längsschnittdarstellung der in **Fig. 19** dargestellten Handhabungseinrichtung;
- **Fig. 21:**: eine Schnittdarstellung der in **Fig. 20** dargestellten Handhabungseinrichtung;
- **Fig. 22:**: eine weitere Ausführungsform einer Handhabungseinrichtung in Längsschnittdarstellung;
- **Fig. 23:**: eine Schnittdarstellung der in **Fig. 22** dargestellten Handhabungseinrichtung;
- **Fig. 24**:: eine isometrische Darstellung einer weiteren Handhabungseinrichtung;
- **Fig. 25:**: eine Explosionsdarstellung der in **Fig. 24** dargestellten Handhabungseinrichtung;
- **Fig. 26:**: eine Schnittdarstellung der in **Fig. 24** dargestellten Handhabungseinrichtung;
- **Fig. 27:**: eine Schnittdarstellung der in **Fig. 26** dargestellten Handhabungseinrichtung gemäß Schnittlinienverlauf XXVII - XXVII;
- **Fig. 28:**: eine Schnittdarstellung der in **Fig. 26** dargestellten Handhabungseinrichtung gemäß Schnittlinienverlauf XXVIII-XXVIII.

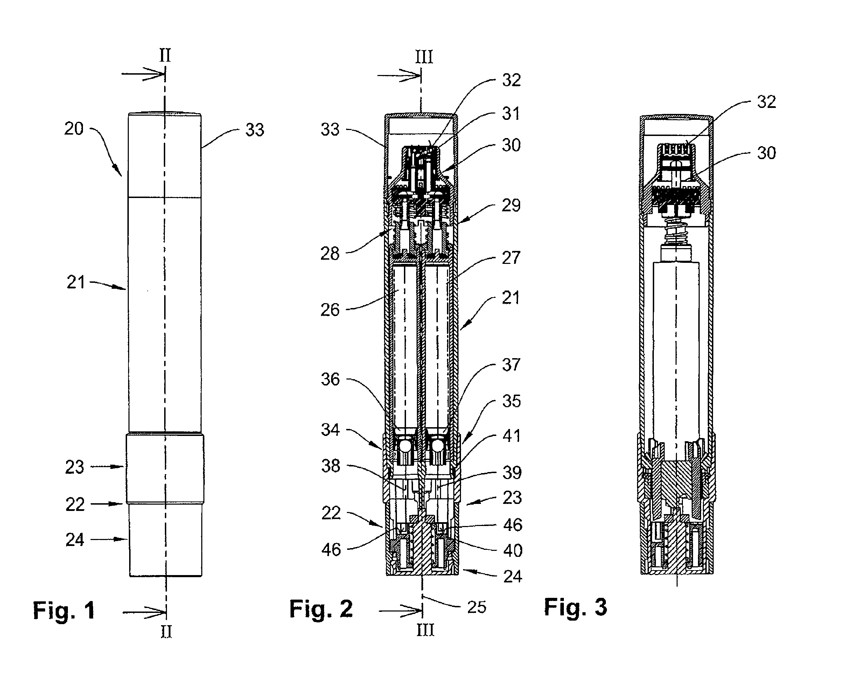

In den **Fig. 1, 2** und **3** ist in einer Gesamtdarstellung ein Dosierspender 20 gezeigt, der als wesentliche Bestandteile eine mit einem Reservoirgehäuse 21 verbundene Handhabungseinrichtung 22 aufweist, die eine Einstelleinrichtung 23 mit einer Betätigungseinrichtung 24 kombiniert, wobei die Stelleinrichtung 23 und die Betätigungseinrichtung 24 jeweils relativ zueinander und gegenüber dem Reservoirgehäuse 21 um eine Längsachse 25 des Dosierspenders 20 drehbar mit dem Reservoirgehäuse 21 verbunden sind.

Wie insbesondere die **Fig. 2** und **3** zeigen, dient das Reservoirgehäuse 21 im Wesentlichen zur Aufnahme zweier Reservoirs 26 und 27, welche im vorliegenden Fall patronenförmig ausgebildet sind und jeweils eine Komponente eines viskosen Stoffs enthalten, bei dem es sich im Falle des hier dargestellten Ausführungsbeispiels des Dosierspenders um einen mit Pigmenten versetzten Lippenpflegestoff handelt, der mittels einer hier oberhalb der Reservoirs 26, 27 im Reservoirgehäuse 21 angeordneten und jeweils einem Reservoir zugeordneten Ventileinrichtung 28 bzw. 29 einer Mischeinrichtung 30 zugeleitet und schließlich aus dieser über eine gemeinsame Ausgabeeinrichtung 31 auf eine Applikatoroberfläche 32 aufgebracht wird, von der die miteinander vermischten Komponenten im Fall des vorliegend als Lippencremeapplikator ausgebildeten Dosierspenders 20 auf die Lippen des Benutzers übertragen werden können. Zur Abdeckung der Applikatoroberfläche 32 bei Nicht-Benutzung des Dosierspenders 20 ist das Reservoirgehäuse 21 mit einer Applikatorkappe 33 versehen.

Wie insbesondere **Fig. 2** zeigt, befinden sich am unteren Ende der Reservoirs 26, 27 Fördereinrichtungen 34, 35, die jeweils unmittelbar Druck auf die in den Reservoirs 26, 27 aufgenommenen Komponenten mittels Förderkolben 36, 37 ausüben, welche über jeweils einen Stößel 38, 39 von einem Übertragungselement 40 beaufschlagt werden, das Bestandteil der Stelleinrichtung 23 der Handhabungseinrichtung 22 ist.

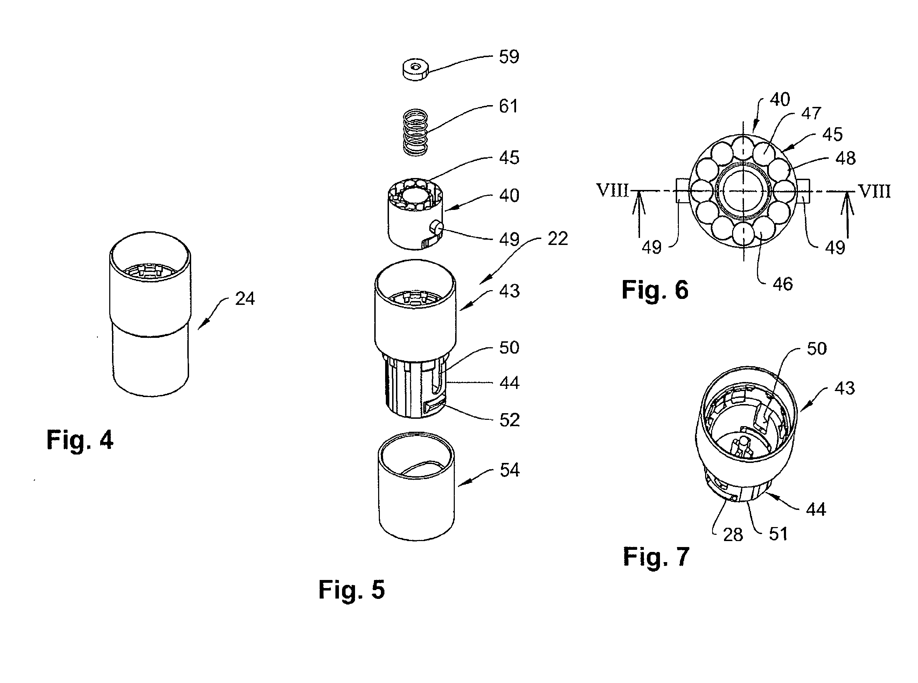

Die in **Fig. 2** und den **Fig. 4 und 5** dargestellte Handhabungseinrichtung 22 ist über eine in **Fig. 2** dargestellte Rastverbindung 41 mit dem Reservoirgehäuse 21 verbunden. Die Stelleinrichtung 23 weist ein Stellgehäuse 43 auf, dessen unterer Teil als Führungsteil 44 zur Aufnahme des Übertragungselements 40 ausgebildet ist. Das Übertragungsteil 40 ist im vorliegenden Fall hülsenartig ausgebildet mit in einer am oberen axialen Ende des Übertragungsteils ringförmig ausgebildeten Kontaktoberfläche 45, die mit einer ringförmig angelegten Reihung von Kontaktabsätzen 46 versehen ist **(****Fig. 6****),** die im vorliegenden Fall durch Böden 47 von in die Kontaktoberfläche 45 eingebrachten Sackbohrungen 48 ausgebildet sind. An seiner Außenwand weist das Übertragungselement 40 zwei diametral zueinander angeordnete Führungszapfen 49 auf, die jeweils in einer als Langloch im Führungsteil 44 des Stellgehäuses 43 ausgebildeten Führungskulisse 50 axial geführt sind.

Wie aus einer Zusammenschau der **Fig. 5, 7** und **10** hervorgeht, weist das Führungsteil 44 des Stellgehäuses 43 an seinem unteren Ende benachbart einem Boden 51 einen Rastrand 52 auf, der einen auf einer Innenwandung 53 eines Betätigungsgehäuses 54 der Betätigungseinrichtung 24 ausgebildeten Raststeg 55 zur Herstellung einer Rastverbindung 56 zwischen dem Betätigungsgehäuse 54 und dem Stellgehäuse 43 hintergreift.

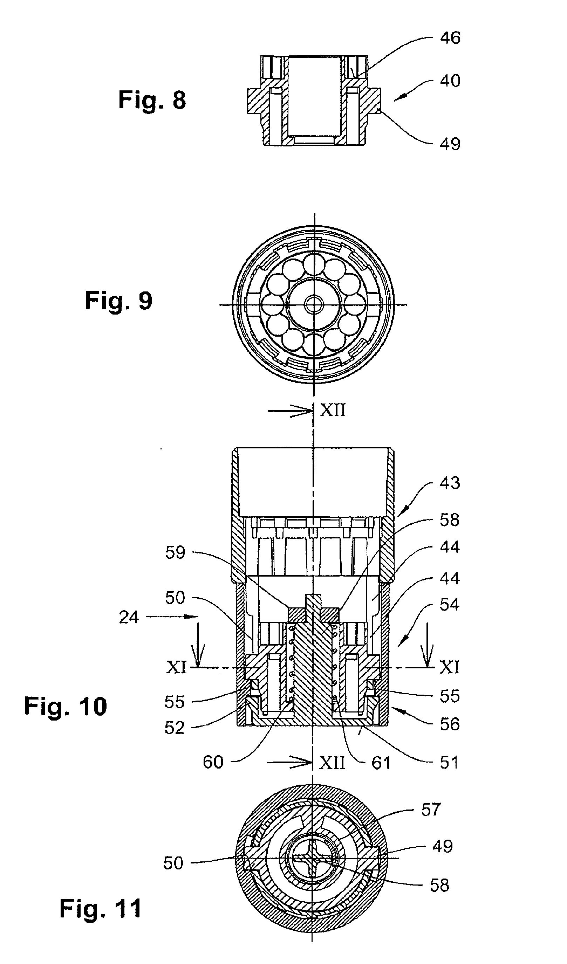

In der insbesondere in **Fig. 10** und **11** dargestellten, verrasteten Stellung zwischen dem Betätigungsgehäuse 54 und dem Stellgehäuse 43 ist das Übertragungselement 40 mit einer zentralen Lagerhülse 57 auf einem am Boden 51 ausgebildeten Zapfen 58 geführt. Der Zapfen 58 ist im Falle des in **Fig. 10** dargestellten Ausführungsbeispiels an seinem oberen Ende mit einem, hier als axialer Sicherungsring ausgebildeten Anschlag 59 versehen, der gegenüberliegend einem Boden 60 der Lagerhülse 57 angeordnet ist und zusammen mit diesem jeweils eine axiale Abstützung einer auf dem Zapfen 58 angeordneten Schraubenfeder 61 bildet. Die in **Fig. 10** dargestellte Ausführung ermöglicht daher eine axiale Verschiebung des Übertragungselements 40 auf dem Zapfen 58 entgegen der durch die Schraubenfeder 61 dabei erzeugten Druckkraft. Bei dieser axialen Verschiebung wird das Übertragungselement 40 mit den Führungszapfen 49 in den Führungskulissen 50 des Führungsteils 44 des Stellgehäuses 43 geführt.

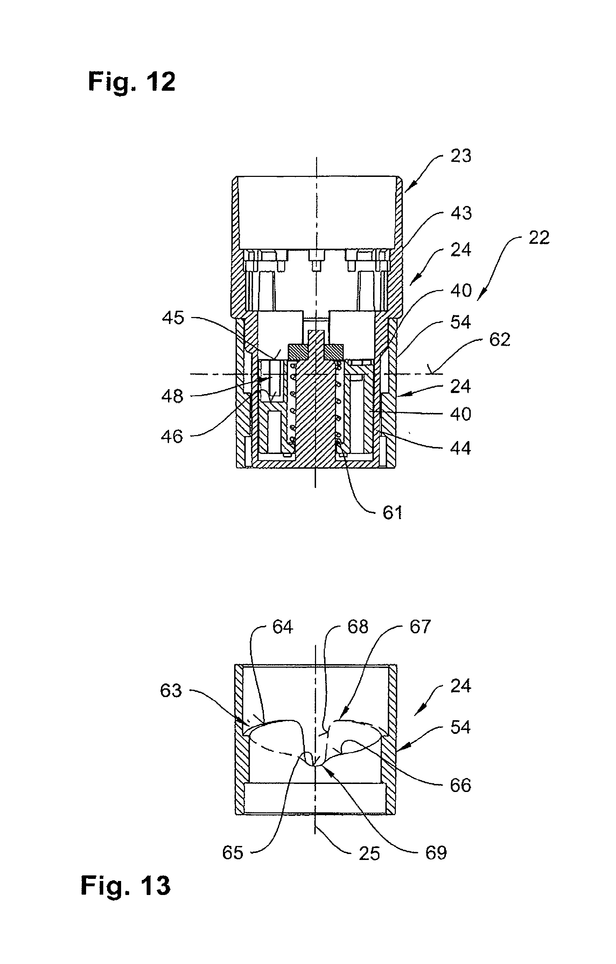

Wie ein Vergleich der **Fig. 10** und **12** zeigt, die die Handhabungseinrichtung 22 in unterschiedlichen Schnittansichten zeigen, weisen die in der Kontaktoberfläche 45 des Übertragungselements 40 zur Ausbildung der Kontaktabsätze 46 angeordneten Sackbohrungen 48 unterschiedliche Tiefen auf, so dass die durch die Böden 47 der Sackbohrungen 48 gebildeten Kontaktabsätze 46 unterschiedliche Niveaus bezogen auf eine mittlere Kontaktebene 62 aufweisen. Abweichend hiervon zeigt Fig. 12 diametral einander gegenüberliegende Kontaktabsätze 46 mit einem gegenüber der mittleren Kontaktflächenebene 62 abweichenden Niveau, so dass bei einer axialen Verstellung des Übertragungselements 40 entgegen der Wirkung der Schraubenfeder 61 bei einem Kontakt der Stößel 38, 39 mit den Kontaktabsätzen 46 die Stößel 38, 39 unterschiedlich weit axial ausgelenkt werden, mit der Folge, dass auch die Förderkolben 36, 37 der Fördereinrichtungen 34, 35 einen unterschiedlichen Hub ausführen.

In den **Fig. 10** und **12** ist das Übertragungselement 40 in voneinander abweichenden Schnittdarstellungen der Handhabungseinrichtung 22 in seiner Ausgangslage dargestellt, in der die Schraubenfeder 61 ihre maximal mögliche Entspannung aufweist und die Führungszapfen 49 am unteren Anschlag der Führungskulissen 50 anliegen. Zur axialen Verstellung des Übertragungselements 40 im Führungsteil 44 ist das Betätigungsgehäuse 54 der Betätigungseinrichtung 24 auf seiner Innenwandung mit einer insbesondere in **Fig. 13** dargestellten Führungsbahn versehen, die hier als ein Führungssteg 63 ausgebildet ist, dessen Kontaktkontur 64 gegen die Führungszapfen 49 des Übertragungselements 40 anliegt **(****Fig. 10****),** so dass bei einer Relativbewegung des Betätigungsgehäuses 54 der Betätigungseinrichtung 24 um die Achse 25 gegenüber dem Stellgehäuse 43 der Stelleinrichtung 23 eine dem Verlauf der Kontaktkontur 64 entsprechende axiale Bewegung des Übertragungselements 40 erfolgt.

Die Kontaktkontur 64 des Führungsstegs 63 weist eine die Ausgangslage des Übertragungselements 40 definierende Vertiefung 65 auf, derart, dass entsprechend der in **Fig. 13** dargestellten Kontaktkontur 64 bei einer Relativbewegung des Betätigungsgehäuses 54 um die Längsachse 25 im Uhrzeigersinn das Übertragungselement 40 entlang einer Aufwärtsrampe 66 axial nach oben bewegt wird. Nach Erreichen eines in **Fig. 13** strichpunktiert angedeuteten Rampenscheitelpunkts 67 bewegt sich dann in Folge der Vorspannkraft der Schraubenfeder 61 das Übertragungsteil 40 entlang einer Abwärtsrampe 68 wieder in eine zweite, der ersten Vertiefung 65 diametral gegenüberliegende Vertiefung 69, wobei dann das Betätigungsgehäuse 54 eine Drehung von 180° um die Längsachse 25 ausgeführt hat.

Bei der vorstehend beschriebenen axialen Vorschubbewegung des Übertragungselements 40 im Stellgehäuse 43 der Stelleinrichtung 23 befindet sich das Stellgehäuse 43 gegenüber dem Reservoirgehäuse 21 in definierter Relativanordnung, so dass sich auch die Drehstellung des Übertragungselements 40 gegenüber den Stößeln 38, 39 nicht verändert.

Wie bereits vorstehend Bezug nehmend auf **Fig. 10** ausgeführt, bewirkt die in **Fig. 2** dargestellte Drehstellung des Übertragungselements, bei der die einander diametral gegenüberliegenden Kontaktabsätze 46 ein übereinstimmendes Niveau aufweisen, eine entsprechend übereinstimmende axiale Verschiebung der Stößel 38, 39. Demzufolge werden durch die Fördereinrichtungen 34, 35 jeweils übereinstimmende Mengen aus den Reservoirs 26, 27 in die Mischeinrichtung 30 und von dieser durch die Ausgabeeinrichtung 31 auf die Applikatoroberfläche 32 gefördert. Zur Einstellung eines hiervon abweichenden Mischungsverhältnisses zwischen den in den Reservoirs 26, 27 aufgenommenen Komponenten kann nun das Stellgehäuse 43 der Stelleinrichtung 23 zusammen mit dem daran angeordneten Betätigungsgehäuse 54 der Betätigungseinrichtung 24 um die Längsachse 25 verdreht werden, bis den Stößeln 38, 39 axial fluchtend Kontaktabsätze 46 zugeordnet sind, die ein voneinander abweichendes Niveau, wie beispielsweise in **Fig. 12** dargestellt, aufweisen, so dass bei einer Kontaktierung dieser Kontaktabsätze 46 in Folge einer nachfolgenden Vorschubbewegung des Übertragungselements gegen die Stößel 38, 39 bei weiterer Drehung des Betätigungsgehäuses 54 gegenüber dem Stellgehäuse 43 die Stößel 38, 39 entsprechend unterschiedlich ausgelenkt werden, mit der weiteren Folge, dass entsprechend unterschiedliche Mengen der Komponenten aus den Reservoirs 26, 27 in die Mischeinrichtung 30 gefördert und auf die Applikatoroberfläche 32 ausgegeben werden.

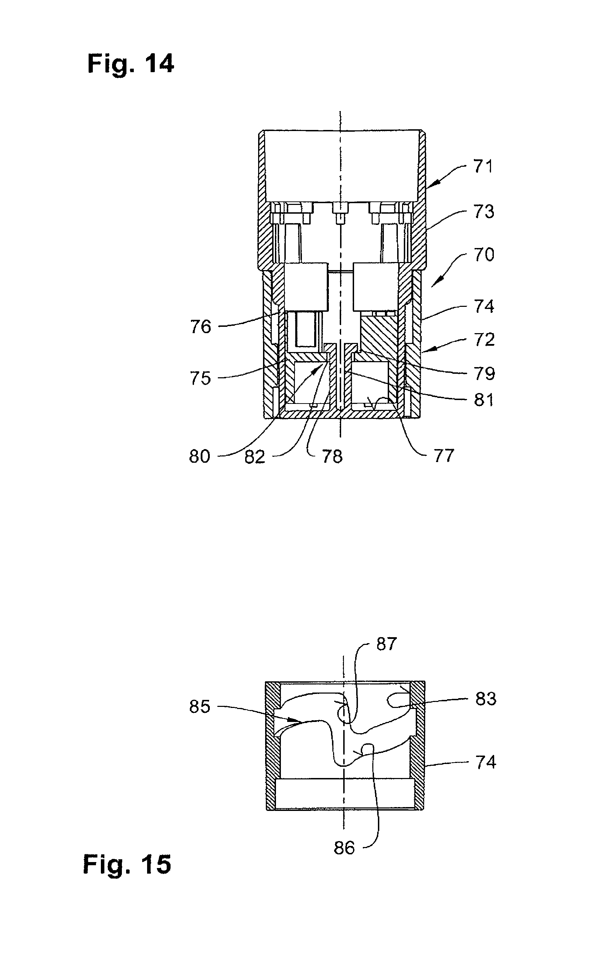

In den **Fig. 14** und **15** ist in einer weiteren Ausführungsform eine Handhabungseinrichtung 70 dargestellt, die eine Stelleinrichtung 71 und eine Betätigungseinrichtung 72 umfasst, welche ein Stellgehäuse 73 bzw. ein Betätigungsgehäuse 74 aufweisen sowie ein Übertragungselement 75, die im Vergleich zu dem Stellgehäuse 43, dem Betätigungsgehäuse 54 und dem Übertragungselement 40 der insbesondere in den **Fig. 10** und **12** dargestellten Handhabungseinrichtung 22 Änderungen aufweisen, die nachstehend erläutert werden sollen.

Zur Definition einer Ausgangsstellung der Betätigungseinrichtung 72 ist in einem Führungsteil 76 des Stellgehäuses 73 ein am Boden 77 angeordneter Rastzapfen 78 vorgesehen, der an seinem axialen Ende eine durch federnd ausgebildete Rastnasen 79 gebildete Rasteinrichtung 80 aufweist, die einen Öffnungsrand 81 einer zentralen Öffnung 82 im Übertragungselement 75 hintergreift.

Zur Führung von in der Ansicht gemäß **Fig. 14** nicht näher dargestellten, jedoch mit den Führungszapfen 49 des Übertragungselements 40 übereinstimmenden Führungszapfen ist in einer Innenwandung 83 des Betätigungsgehäuses 74 eine Führungsbahn als Führungsnut 85 ausgebildet mit parallel zueinander verlaufenden Nuträndern 86, 87, die die Führungszapfen zwischen sich aufnehmen und so sowohl bei einer Aufwärtsbewegung als auch bei einer Abwärtsbewegung des Übertragungselements 75 für einen sicheren Kraftschluss zwischen der durch die Führungsnut 85 gebildeten Führungseinrichtung des Betätigungsgehäuses 74 und dem Übertragungselement 75 sorgen.

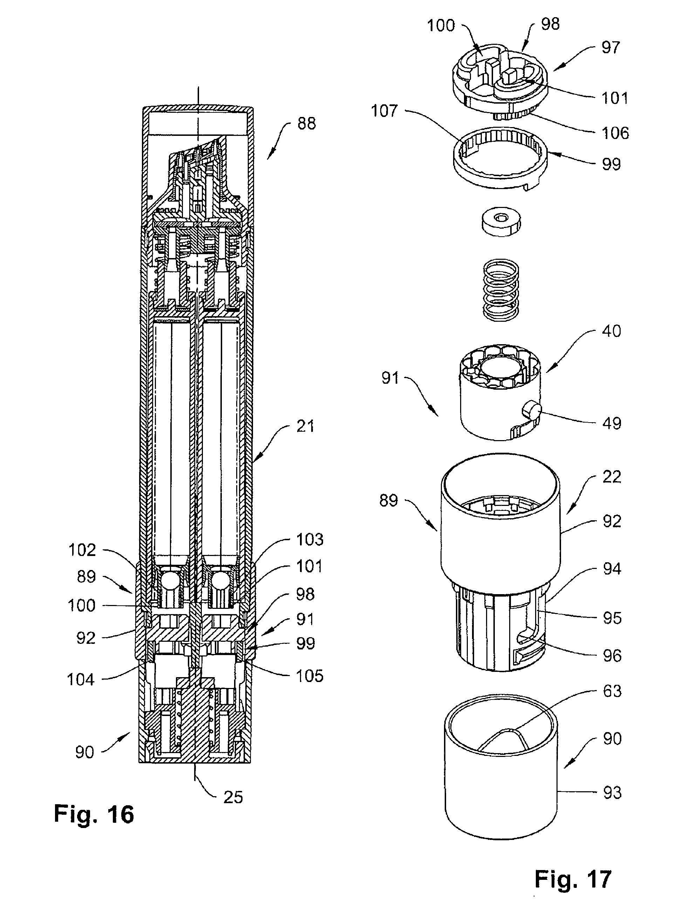

**Fig. 16** zeigt einen Dosierspender 88, der analog dem in den **Fig. 1** bis **3** dargestellten Dosierspender 20 mit einem Reservoirgehäuse 21 versehen ist, das mit einer Stelleinrichtung 89 und einer Betätigungseinrichtung 90 versehen ist, die eine Handhabungseinrichtung 91 ausbilden und auf einer gemeinsamen Längsachse 25 mit dem Reservoirgehäuse 21 angeordnet sind.

Die Handhabungseinrichtung 91 ist in **Fig. 17** in einer Explosionsdarstellung gezeigt und umfasst ein Stellgehäuse 92 der Stelleinrichtung 89 sowie ein Betätigungsgehäuse 93 der Betätigungseinrichtung 90. Ein Vergleich mit der in **Fig. 5** dargestellten Handhabungseinrichtung 22 des Dosierspenders 20 zeigt, dass das Stellgehäuse 92 mit einem Führungsteil 94 versehen ist, der im Unterschied zu dem Führungsteil 44 des Stellgehäuses 43 mit einer Führungskulisse 95 versehen ist, die an ihrem unteren Ende eine hier als Ausnehmung ausgebildete Zapfenfalle 96 aufweist. Die Zapfenfalle 96 wirkt mit dem in der Führungskulisse 95 geführten Führungszapfen 49 des Übertragungselements 40 derart zusammen, dass bei einer Drehung des Betätigungsgehäuses 93 der Betätigungseinrichtung 90 im Uhrzeigersinn das Übertragungselement 40 durch den Führungssteg 63 auf der Längsachse 25 nach oben bewegt wird. Bei einer Drehung des Betätigungsgehäuses 93 entgegen dem Uhrzeigersinn wird jedoch eine translatorische Aufwärtsbewegung des Übertragungselements 40 auf der Längsachse 25 dadurch blockiert, dass der Führungszapfen 49 in der Zapfenfalle 96 gehalten wird. Die derart ausgebildete Führungskulisse 95 des Stellgehäuses 92 definiert somit eine Betätigungs- bzw. Drehrichtung für die Betätigungseinrichtung 90.

Als weiterer Unterschied zu der Handhabungseinrichtung 22 des Dosierspenders 20 weist die Handhabungseinrichtung 91 des Dosierspenders 88 eine modular ausgebildete Rasteinrichtung 97 auf, die zwei voneinander unabhängig ausgebildete ringförmige Rastelemente 98, 99 aufweist, welche zwischen dem Reservoirgehäuse 21 und dem Stellgehäuse 92 derart angeordnet sind, dass das Rastelement 98 über Oberflächenausnehmungen 100, 101, in die Fortsätze 102 , 103 des Reservoirgehäuses 21 eingreifen, drehfest mit dem Reservoirgehäuse 21 verbunden ist. Das Rastelement 99 weist Klinkenfortsätze 104, 105 auf, über die das Rastelement 99 drehfest mit dem Stellgehäuse 92 verbunden ist. Zwischen dem Rastelement 98 und dem Rastelement 99 ist ein Rasteingriff hergestellt durch eine jeweils am Rastelement 98 sowie am Rastelement 99 ausgebildete Sperrklinken-Verzahnung 106, 107, die eine Relativdrehung der Rastelemente 98, 99 bei der in **Fig. 17** dargestellten Ausführungsform mit geneigten Zahnflanken 108 nur entgegen dem Uhrzeigersinn ermöglicht, so dass bei der in den **Fig. 16** und **17** dargestellten Ausführungsform des Dosierspenders 88 die Betätigungseinrichtung 90 im Uhrzeigersinn und die Stelleinrichtung 89 entgegen dem Uhrzeigersinn betätigt werden können.

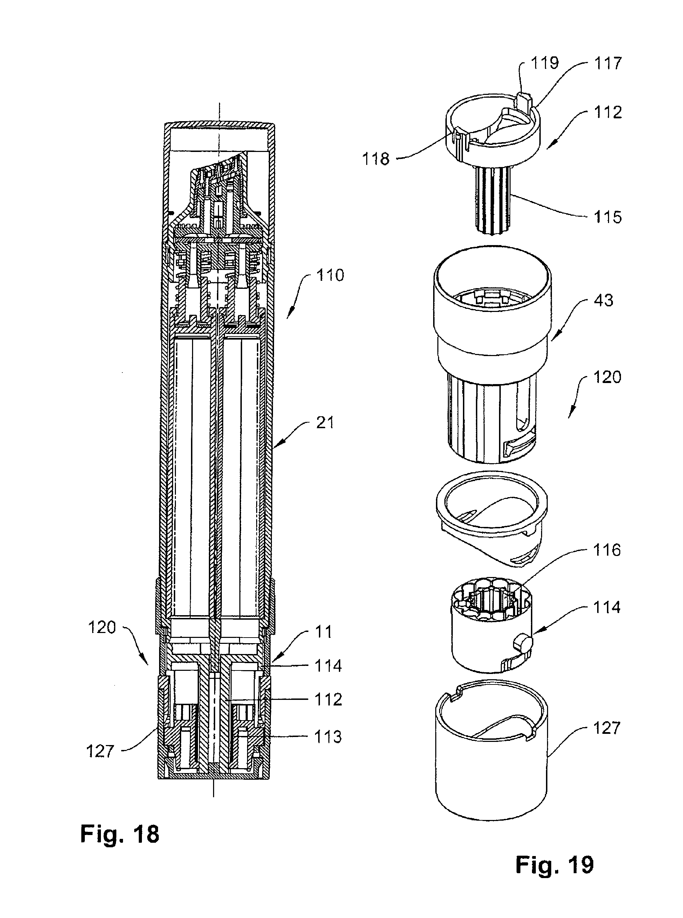

In den **Fig. 18** und **19** ist in einer weiteren Ausführungsform ein Dosierspender 110 mit einer Handhabungseinrichtung 120 dargestellt, der im Unterschied zu dem vorstehend erläuterten Dosierspender 88 eine Rasteinrichtung 111 aufweist, die eine mit dem Reservoirgehäuse 21 drehfest verbundene Rastachse 112 aufweist, welche in eine als Rasthülse 113 ausgebildete Nabe eines Übertragungselements 114 eingreift und dabei einen Rasteingriff zwischen einer auf dem Außenumfang der Rastachse 112 ausgebildeten Rastverzahnung 115 und einer auf der Bohrungswandung der Rasthülse 113 ausgebildeten Rastverzahnung 116 ausbildet. Die Rastachse 112 ist mit einer tellerförmigen Achsenbasis 117 versehen, die an ihrem oberen Rand Vorsprünge 118, 119 zum Eingriff in nicht näher dargestellte, am unteren Rand des Reservoirgehäuses 21 ausgebildete Ausnehmungen aufweist und eine drehfeste Verbindung zum Reservoirgehäuse 21 herstellt.

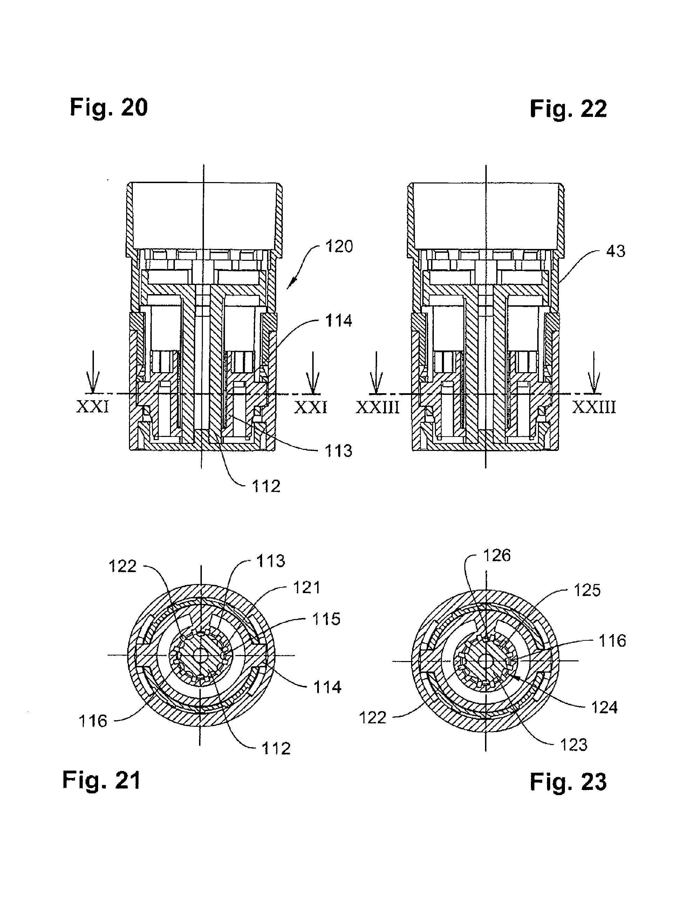

**Fig. 21** zeigt einen Querschnitt der in **Fig. 20** dargestellten Handhabungseinrichtung 120 mit Darstellung der in die Rasthülse 113 des Übertragungselements 114 eingreifenden Rastachse 112, wobei in dem dargestellten Ausführungsbeispiel die Rastverzahnung 115 der Rastachse 112 im Querschnitt symmetrisch ausgebildete Rastzähne 121 aufweist, die in die durch Rastnuten 122 ausgebildete Rastverzahnung 116 des Übertragungselements 114 eingreifen. Aufgrund der symmetrischen Ausgestaltung der Rastzähne 121 ermöglicht der Rasteingriff zwischen der Rastachse 112 und der Rasthülse 113 eine Relativdrehung sowohl im Uhrzeigersinn als auch entgegen dem Uhrzeigersinn.

Im Vergleich dazu weist eine in **Fig. 23** dargestellte Rastachse 123 eine Rastverzahnung 124 mit Rastzähnen 125 auf, die sperrklinkenartig ausgebildet sind mit geneigten Zahnflanken 126, die durch die Rastnuten 122 der Rastverzahnung 116 des Übertragungselements 1 14 eine Relativdrehung zwischen dem Stellgehäuse 43 **(****Fig. 19****)** und dem Reservoirgehäuse 21 (siehe **Fig. 18****)** lediglich entgegen dem Uhrzeigersinn ermöglichen.

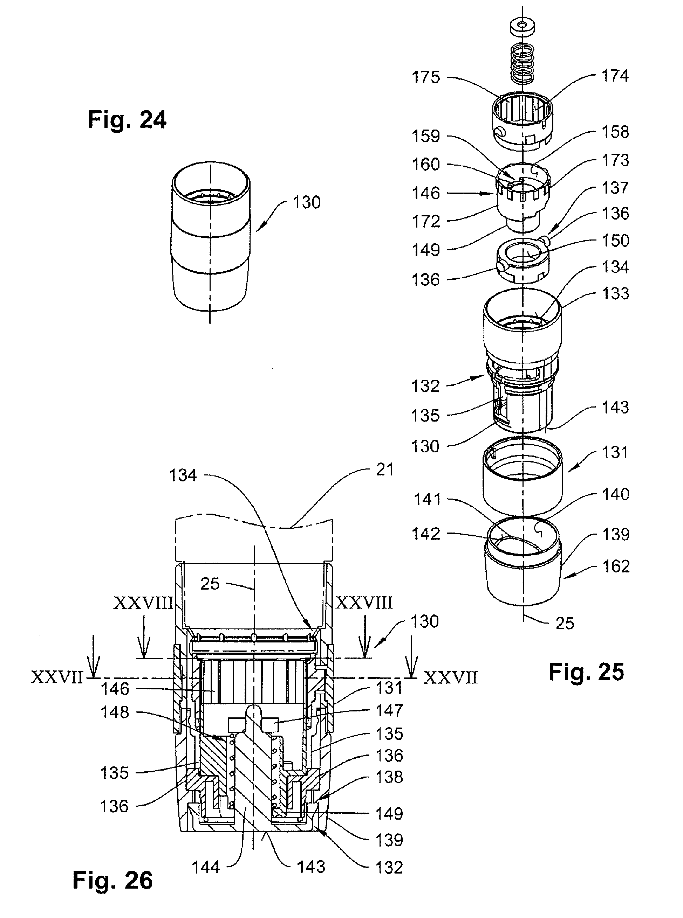

In den **Fig. 24** bis **26** ist in einer weiteren Ausführungsform eine Handhabungseinrichtung 130 dargestellt, die, wie die vorstehend bereits beschriebenen Handhabungseinrichtungen 22, 42, 70, 91 und 120 mit dem Reservoirgehäuse 21 kombinierbar ist, wobei im Unterschied zu den bereits beschriebenen Handhabungseinrichtungen 22, 42, 70, 91 und 120 die Handhabungseinrichtung 130 ein unabhängig von einem Stellgehäuse 131 ausgebildetes Führungsteil 132 aufweist, das als Führungsgehäuse ausgebildet ist und über einen an seinem oberen Ende ausgebildeten Befestigungsring 133 mittels einer Rastverbindung 134 drehfest mit dem Reservoirgehäuse 21 verbunden ist.

Wie insbesondere eine Zusammenschau der **Fig. 25** und **26** deutlich macht, ist das Führungsteil 132 mit Führungskulissen 135 versehen, in denen jeweils ein Führungszapfen 136 einer als Führungsring 137 ausgebildeten Zapfenanordnung axial geführt aufgenommen wird.

Weiterhin zeigt insbesondere die **Fig. 25****,** dass am unteren Ende des Führungsteils 132 eine Rastverbindung 138 zwischen dem Führungsteil 132 und einem auf dem Führungsteil 132 drehbar angeordneten Betätigungsgehäuse 139 einer Betätigungseinrichtung 162 ausgebildet ist. Das Betätigungsgehäuse 139 weist auf seiner Innenwandung 140 einen Führungssteg 141 auf, der eine mit definierter Steigung ausgebildete Kontaktkontur 142 aufweist, derart, dass bei einer Relativdrehung des Betätigungsgehäuses 139 auf dem Führungsteil 132 die Führungszapfen 136 auf der Kontaktkontur 142 entlang gleiten und dabei in den Führungskulissen 135 des Führungsteils 132 eine axiale Bewegung ausführen, die je nach Drehrichtung des Betätigungsgehäuses 139 aufwärts oder abwärts gerichtet ist, mit der Folge, dass der Führungsring 137 eine entsprechende Aufwärts- oder Abwärtsbewegung ausführt.

Auf einem an einem Boden 143 des Führungsteils 132 ausgebildeten Zapfen 144, ist eine Schraubenfeder 148 angeordnet, die sich mit einem Ende an einen Boden 145 eines im Führungsteil 132 aufgenommenen Übertragungselementes 146 und mit dem anderen Ende an einem auf dem Zapfen 144 angeordneten Anschlag 147 abstützt. Die Schraubenfeder 148 sorgt somit dafür, dass das Übertragungselement 146 gegen den Führungsring 137 anliegt, wobei ein becherförmiger Vorsprung 149 des Übertragungselements 146 in eine Ringöffnung 150 des Führungsrings 137 eingreift **(****Fig. 25****).** Gleichzeitig sorgt die Schraubenfeder 148 dafür, dass, unabhängig davon, ob der Führungsring 137 eine Aufwärtsbewegung oder Abwärtsbewegung im Führungsteil 132 ausführt, die Führungszapfen 136 des Führungsrings 137 definiert auf der Kontaktkontur 142 aufliegen.

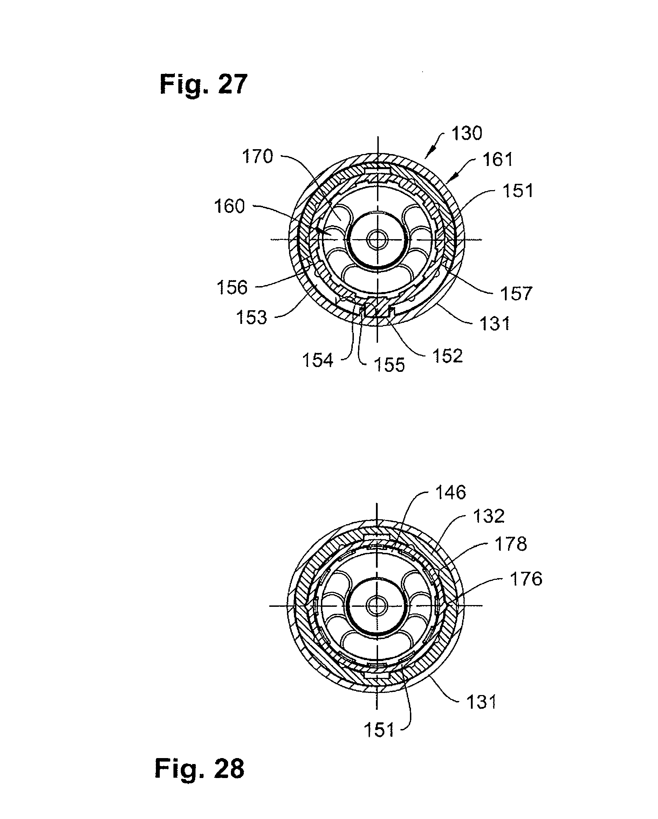

Wie insbesondere eine Zusammenschau der **Fig. 26** und **27** verdeutlicht, ist bei der Handhabungseinrichtung 130 eine Stelleinrichtung 161, die zur definierten Verstellung bzw. Verdrehung des Übertragungselements 146 auf der Längsachse 25 der Handhabungseinrichtung 130 dient, aus mehreren Teilen aufgebaut und umfasst neben dem hier ring- oder hülsenförmig ausgebildeten Stellgehäuse 131 eine konzentrisch zum Stellgehäuse 131 innerhalb des Führungsteils 132 angeordnete Stellhülse 151. Die Stellhülse 151 ist mit einem radial nach außen ragenden Stellzapfen 152 versehen, der eine im Führungsteil 132 ausgebildete Stellnut 153 durchdringt und durch einen Eingriff in eine auf eine Innenwandung 154 des Stellgehäuses 131 ausgebildete Führungstasche 155 eine drehfeste Verbindung mit dem Stellgehäuse 131 herstellt, derart, dass bei einer Drehung des Stellgehäuses 131 um die Längsachse 25 der Handhabungseinrichtung 130 die Stellhülse 151 zwangsweise mitgedreht wird. Dabei wird der radiale Stellweg, den der Stellzapfen 152 ausführt, durch radiale Anschläge 156, 157 begrenzt, die durch die Stellnut 153 definiert werden. Wie ferner insbesondere eine Zusammenschau der **Fig. 25** und **27** deutlich macht, ist das Übertragungselement 146 auf seiner Innenwandung 158 mit einer Kontaktoberfläche 159 versehen ist, die in einem Ringsegment 160 Kontaktabsätze 170 aufweist, die ausgehend von einem tiefliegenden, eine Mittellage definierenden mittleren Kontaktabsatz 170 längs des Umfangs in beide Richtungen ansteigend angeordnet sind.

Wie insbesondere **Fig. 25** zeigt, weist das Übertragungselement 146 an seiner Außenwandung 172 Mitnehmervorsprünge 173 auf, die in auf einer Innenwandung 174 der Stellhülse 151 ausgebildete Mitnehmeraufnahmen 175 eingreifen. Dadurch wird das Übertragungselement 146 innerhalb der Stellhülse 151 axial verstellbar und bezogen auf die Längsachse 25 der Handhabungseinrichtung 130 drehfest in der Stellhülse 151 aufgenommen, derart, dass bei einer Drehung des äußeren Stellgehäuses 131 um die Längsachse 25 das Übertragungselement 146 eine entsprechende Drehung um die Längsachse 25 ausführt.

Zur definierten Relativanordnung des Stellgehäuses 131 bzw. des Übertragungselements 146 und dem drehfest mit dem Reservoirgehäuse 21 verbundenen Führungsteil 132 ist zwischen der Stellhülse 151 und dem Führungsteil 132 eine in **Fig. 28** dargestellte Rasteinrichtung 171 vorgesehen, derart, dass eine an der Außenwand der Stellhülse 151 ausgebildete Rastnase 176 mit einer Mehrzahl von umlaufend in der Innenwandung des Führungsteils 132 ausgebildeten Rastfallen 178 zusammenwirkt, wobei durch Überwindung eines eine definierte Rastposition bestimmenden Drehwiderstands zwischen der Stellhülse 151 und dem Führungsteil 132 einer definierten Drehstellung, in der die Rastnase 176 in eine erste Rastfalle 178 eingreift, in eine weitere definierte Drehstellung, in der die Rastnase 176 in eine andere Rastfalle 178 eingreift, gewechselt werden kann.

Aufgrund der vorteilhaften Führung bzw. Aufnahme des Übertragungselements 146 im Führungsring 137 wird bei einer axialen Verstellung des Führungsrings 137 innerhalb des Führungsteils 132 in Folge einer Verdrehung des Betätigungsgehäuses 139 gegenüber dem drehfest am Reservoirgehäuse 21 angeordneten Führungsteil 132 kein Drehmoment auf das Übertragungselement 146 übertragen, sodass auch kein Drehmoment vom Übertragungselement 146 auf die beispielsweise in der **Fig. 2** dargestellten Stößel 38, 39 der Fördereinrichtungen 34, 35 wirken kann, das ein Bauteilversagen der Stößel 38, 39 verursachen könnte.

## Patentansprüche

1. Dosierspender (20, 88, 110) zur dosierten Abgabe von zumindest zwei in jeweils einem Reservoir (26, 27) aufgenommenen Komponenten, wobei jedem Reservoir (26, 27) eine Fördereinrichtung (34, 35) zugeordnet ist und die Fördereinrichtungen mit einer Betätigungseinrichtung (24, 72, 90, 162) zur Ausgabe der Komponenten aus einer mit den Reservoirs (26, 27) verbundenen Ausgabeeinrichtung (31) versehen sind, wobei die Betätigungseinrichtung mittelbar über eine Dosiereinrichtung auf die Fördereinrichtungen wirkt und die Dosiereinrichtung mit einer Stelleinrichtung (23, 71, 89, 161) versehen ist, mit deren Betätigung die Stellung eines auf die Fördereinrichtungen wirkenden Übertragungselements (40, 75, 114, 146) zur Einstellung des Mengenverhältnisses der Komponenten veränderbar ist,
**dadurch gekennzeichnet,**
**dass** sowohl die Stelleinrichtung (23, 71, 89, 161) zur Einstellung des Mengenverhältnisses der Komponenten als auch die Betätigungseinrichtung (24, 72, 90, 162) zur Ausgabe der Komponenten aus der Ausgabeeinrichtung (31) zu ihrer Betätigung um eine Längsachse (25) des Dosierspenders (20, 88, 110) drehbar sind.

2. Dosierspender nach Anspruch 1,
**dadurch gekennzeichnet,**
**dass** die Stelleinrichtung (23, 71, 89, 161) der Dosiereinrichtung ein um die Längsachse (25) eines Reservoirgehäuses(21), in dem die Reservoire (26, 27) zur Aufnahme der Komponenten und die Fördereinrichtungen (34, 35) aufgenommen sind, drehbares Stellgehäuse (43, 73, 92, 131) aufweist, in dem das Übertragungselement (40, 75, 114, 146) gegenüber dem Stellgehäuse drehstarr und auf der Längsachse des Stellgehäuses zur Betätigung der Fördereinrichtung axial verschiebbar angeordnet ist, wobei das Übertragungselement eine ringförmig oder als Ringsegment (160) ausgebildete Kontaktoberfläche (45, 139) aufweist, mit einer sich umlaufend ändernden Oberflächenkontur, die mit Förderkolben (36, 37) der Fördereinrichtungen zusammenwirkt.

3. Dosierspender nach Anspruch 2,
**dadurch gekennzeichnet,**
**dass** die Betätigungseinrichtung (24, 72, 90, 162) zur Ausgabe der Komponenten ein um die Längsachse (25) des Dosierspenders (20) relativ zum Stellgehäuse (43, 73, 92, 131) drehbares Betätigungsgehäuse (54, 74, 93, 139) aufweist, das mit einer Führungsanordnung zur axialen Verschiebbarkeit des Übertragungselementes (40, 75, 114, 146) versehen ist, wobei eine erste im Betätigungsgehäuse ausgebildeten Führungseinrichtung der Führungsanordnung zur Umwandlung einer Drehbewegung des Betätigungsgehäuses in eine axiale Bewegung des Übertragungselements mit einer zweiten unabhängig vom Betätigungsgehäuse ausgebildeten Führungseinrichtung der Führungsanordnung zusammenwirkt.

4. Dosierspender nach Anspruch 3,
**dadurch gekennzeichnet,**
**dass** die zweite Führungseinrichtung ein Führungsteil (44, 76, 94, 132) mit einer axial ausgerichteten Führungskulisse (50, 95, 135) und eine am Übertragungselement (40, 75, 114, 146) positionierte Zapfenanordnung mit zumindest einem radialen Führungszapfen (49, 136) aufweist, der die Führungskulisse durchdringt und mit seinem Kontaktende mit der ersten Führungseinrichtung des Betätigungsgehäuses (54, 74, 93, 139) zusammenwirkt.

5. Dosierspender nach Anspruch 3 oder 4,
**dadurch gekennzeichnet,**
**dass** das Betätigungsgehäuse (54, 74, 93, 139) zur Ausbildung der ersten Führungseinrichtung auf seiner koaxial zum Führungsteil (44, 76, 94, 132) angeordneten Innenwandung (51, 83, 140) eine Führungsbahn mit einer mit dem Kontaktende des Führungszapfens (49, 136) zusammenwirkenden Kontaktkontur (64, 142) aufweist, die die axiale Bewegung des Übertragungselements (40, 75, 114, 146) steuert.

6. Dosierspender nach Anspruch 4 oder 5,
**dadurch gekennzeichnet,**
**dass** die im Führungsteil (94, 132) ausgebildete, axial ausgerichtete Führungskulisse (95, 135) an ihrem unteren Ende zur Definition einer Drehrichtung der Betätigungseinrichtung (90, 162) eine Zapfenfalle (96) zur Aufnahme des radialen Führungszapfens (49, 136) aufweist.

7. Dosierspender nach einem der vorangehenden Ansprüche,
**dadurch gekennzeichnet,**
**dass** zur Definition einer Ausgangsstellung der Betätigungseinrichtung (24, 162), in der die Fördereinrichtungen (34, 35) nicht durch das Übertragungselement (40, 146) beaufschlagt werden, zwischen einem am Führungsteil (44, 132) ausgebildeten Anschlag (59, 147) und dem Übertragungselement (40, 146) eine Federeinrichtung angeordnet ist.

8. Dosierspender nach Anspruch 7,
**dadurch gekennzeichnet,**
**dass** der Anschlag (59, 147) am axialen Ende eines am Boden des Führungsteils (44, 132) angeordneten Zapfens (58, 144) ausgebildet ist, der durch eine in einem Boden (60, 143) einer zentralen becherförmigen Vertiefung des Übertragungselements (40, 146) ausgebildete Öffnung erstreckt, und dass die Federeinrichtung als eine Schraubenfeder (61, 148) ausgebildet ist, die in einem zwischen dem Zapfen und der Vertiefung ausgebildeten Ringraum angeordnet ist und sich zwischen dem Anschlag und dem Boden erstreckt.

9. Dosierspender nach einem der Ansprüche 1 bis 6,
**dadurch gekennzeichnet,**
**dass** zur Definition einer Ausgangsstellung der Betätigungseinrichtung (72), in der die Fördereinrichtungen nicht durch das Übertragungselement (75) beaufschlagt werden, an einem axialen Ende eines im Führungsteil (76) angeordneten Rastzapfens (78) eine Rasteinrichtung (80) angeordnet ist, die bei dem in Ausgangstellung befindlichen Übertragungselement (75) einen Rand einer zentralen Öffnung (82) im Übertragungselement (75) hintergreift.

10. Dosierspender nach einem der vorangehenden Ansprüche,
**dadurch gekennzeichnet,**
**dass** die in der Innenwandung (83) des Betätigungsgehäuses (74) angeordnete Führungsbahn als Führungsnut (85) ausgebildet ist, deren parallel zueinander verlaufenden Nutränder (86, 87) den Führungszapfen (49) zwischen sich aufnehmen.

11. Dosierspender nach einem der vorangehenden Ansprüche,
**dadurch gekennzeichnet,**
**dass** zur Ausbildung der zweiten Führungseinrichtung das Führungsteil (44, 76, 94) durch das Stellgehäuse (43, 73, 92) ausgebildet ist, und die Zapfenanordnung mit dem zumindest einen Führungszapfen (49) am Übertragungselement (40, 75, 114) ausgebildet ist, derart, dass bei einer Drehung des Stellgehäuses eine entsprechende Drehung des Übertragungselements erfolgt.

12. Dosierspender nach einem der vorangehenden Ansprüche,
**dadurch gekennzeichnet,**
**dass** zur definierten Relativanordnung des Stellgehäuses (43, 73, 92) zum Reservoirgehäuse (21) eine Rasteinrichtung (41, 97, 111) zwischen dem Stellgehäuse (43, 73, 92) und dem Reservoirgehäuse (21) ausgebildet ist, die eine Mehrzahl von Raststellungen für eine definierte Zuordnung von auf der Kontaktoberfläche (45) ausgebildeten Kontaktstellen des Übertragungselements (40, 75, 114) und auf Förderkolben (36, 37) der Fördereinrichtungen (34, 35) wirkenden Stößeln (38, 39) aufweist.

13. Dosierspender nach Anspruch 12,
**dadurch gekennzeichnet,**
**dass** die Rasteinrichtung (97) als eine modulare, zwischen das Reservoirgehäuse (21) und das Stellgehäuse (92) einsetzbare Rasteinheit ausgebildet ist, die drehrichtungsabhängig zur Relativdrehung des Stellgehäuses gegenüber dem Reservoirgehäuse betätigbar ist.

14. Dosierspender nach Anspruch 13,
**dadurch gekennzeichnet,**
**dass** die Rasteinheit ein erstes, drehfest mit dem Reservoirgehäuse (21) verbindbares, ringförmiges Rastelement (98) und ein zweites, drehfest mit dem Stellgehäuse (92) verbindbares, ringförmiges Rastelement (99) aufweist, wobei die Rastelemente über einen in einer gemeinsamen Ringebene ausgebildeten, durch Rastvorsprünge hergestellten Rasteingriff zusammenwirken, und die Rastvorsprünge durch eine Sperrklinken-Verzahnung (106, 107) ausgebildet sind.

15. Dosierspender nach Anspruch 14,
**dadurch gekennzeichnet,**
**dass** eines der beiden Rastelemente (98, 99) die Sperrklinken-Verzahnung (106, 107) lediglich in einem Ringsegment aufweist.

16. Dosierspender nach Anspruch 12,
**dadurch gekennzeichnet,**
**dass** die Rasteinrichtung (111) eine mit dem Reservoirgehäuse (21) verbundene Rastachse (112) aufweist, die zur Ausbildung eines Rasteingriffs in eine als Rasthülse (113) ausgebildete Nabe des Übertragungselements (114) eingreift.

17. Dosierspender nach Anspruch 16,
**dadurch gekennzeichnet**
**dass** zur Ausbildung des Rasteingriffs auf dem Umfang der Rastachse (112) ausgebildete Rastvorsprünge mit auf der Bohrungswandung der Rasthülse (113) ausgebildeten Rastvorsprüngen zusammenwirken.

18. Dosierspender nach Anspruch 17,
**dadurch gekennzeichnet,**
**dass** die Rastvorsprünge durch eine Sperrklinken-Verzahnung (124) ausgebildet sind.

19. Dosierspender nach einem der Ansprüche 1 bis 10,
**dadurch gekennzeichnet,**
**dass** zur Ausbildung der zweiten Führungseinrichtung das Führungsteil (132) drehfest mit dem Reservoirgehäuse (21) verbunden ist, die Zapfenanordnung als Führungsring (137) mit zumindest einem Führungszapfen (136) ausgebildet ist, und das Übertragungselement (146) im Führungsring drehbar aufgenommen und mittels einer radialen Eingriffseinrichtung drehfest mit dem Stellgehäuse (131) verbunden ist, derart, dass bei einer Drehung des Stellgehäuses dieses eine Relativdrehung gegenüber dem Führungsteil ausführt.

20. Dosierspender nach Anspruch 19,
**dadurch gekennzeichnet,**
**dass** die Eingriffseinrichtung eine Stellhülse (151) aufweist, in der das Übertragungselement (146) drehfest und axial verschiebbar aufgenommen ist, wobei die Stellhülse über einen Stellzapfen (152), der das Führungsteil (132) in einer Stellnut (153) radial durchdringt, eine Eingriffsverbindung mit dem Stellgehäuse (131) ausbildet.

21. Dosierspender nach Anspruch 20,
**dadurch gekennzeichnet,**
**dass** zur definierten Relativanordnung des Stellgehäuses (131) eine Rasteinrichtung (171) zwischen der Stellhülse (151) und dem Führungsteil (132) ausgebildet ist, die eine Mehrzahl von Raststellungen für eine definierte Zuordnung von auf der Kontaktoberfläche (159) ausgebildeten Kontaktstellen des Übertragungselements (146) und auf Förderkolben (36, 37) der Fördereinrichtungen (34, 35) wirkenden Stößeln (38, 39) aufweist.

22. Dosierspender nach einem der vorangehenden Ansprüche,
**dadurch gekennzeichnet,**
**dass** die Kontaktstellen auf der Kontaktoberfläche (45, 159) des Übertragungselements (40, 75, 146) durch in einer Stufenfolge angeordnete Kontaktabsätze (46, 170) gebildet sind.

23. Dosierspender nach Anspruch 22,
**dadurch gekennzeichnet,**
**dass** die Kontaktabsätze (46) durch Sackbohrungen (48) ausgebildet sind, die in einer horizontalen Oberfläche des Übertragungselements (40, 75) angeordnet sind.

## Claims

1. Dispenser (20, 88, 110) for the dosed dispensing of at least two components received in one reservoir (26, 27) respectively, wherein a pumping device (34, 35) is assigned to each reservoir (26, 27) and the pumping devices are equipped with an actuation device (24, 72, 90, 162) for dispensing the components from an output device (31) which is connected to the reservoirs (26, 27), wherein the actuation device acts on the pumping devices indirectly via a dosing device and the dosing device is equipped with a setting device (23, 71, 89, 161) which can be actuated to change the position of a transmission element (40, 75, 114, 146) acting on the pumping devices for setting the quantity ratio of the components,
**characterized in that**
both the setting device (23, 71, 89, 161) for setting the quantity ratio of the components and the actuation device (24, 72, 90, 162) for dispensing the components from the output device (31), for the purpose of actuation, can be rotated about a longitudinal axis (25) of the dispenser (20, 88, 110).

2. Dispenser according to Claim 1,
**characterized in that**
the setting device (23, 71, 89, 161) of the dosing device has an adjusting housing (43, 73, 92, 131) which can be rotated about the longitudinal axis (25) of a reservoir housing (21), in which the reservoirs (26, 27) for receiving the components and the pumping devices (34, 35) are received, and in which adjusting housing the transmission element (40, 75, 114, 146) is arranged in a rotationally fixed manner with respect to the adjusting housing and so as to be axially displaceable on the longitudinal axis of the adjusting housing for actuating the pumping device, wherein the transmission element has a contact surface (45, 159) of an annular design or being formed as a ring segment (160) which has a surface contour changing in shape along its circumference and interacting with pumping pistons (36, 37) of the pumping devices.

3. Dispenser according to Claim 2,
**characterized in that**
the actuation device (24, 72, 90, 162) for dispensing the components has an actuating housing (54, 74, 93, 139) which can be rotated about the longitudinal axis (25) of the dispenser (20) relative to the adjusting housing (43, 73, 92, 131), said actuating housing being equipped with a guide arrangement to enable the axial displacement of the transmission element (40, 75, 114, 146), wherein a first guide device of the guide arrangement, which is formed in the actuating housing, interacts with a second guide device of the guide arrangement, which is formed independently of the actuating housing, for converting a rotary movement of the actuating housing into an axial movement of the transmission element.

4. Dispenser according to Claim 3,
**characterized in that**
the second guide device has a guide section (44, 76, 94, 132) having an axially oriented guide slot (50, 95, 135) and a pin arrangement positioned at the transmission element (40, 75, 114, 146) with at least one radial guide pin (49, 136) which penetrates through the guide slot and with its contact end interacts with the first guide device of the actuating housing (54, 74, 93, 139).

5. Dispenser according to Claim 3 or 4,
**characterized in that**
for the purpose of forming the first guide device, the actuating housing (54, 74, 93, 139), on its inner wall (51, 83, 140) being coaxially arranged with respect to the guide section (44, 76, 94, 132), has a guide path having a contact contour (64, 142) interacting with the contact end of the guide pin (49, 136) and controlling the axial movement of the transmission element (40, 75, 114, 146).

6. Dispenser according to Claim 4 or 5,
**characterized in that**
the axially oriented guide slot (95, 135) formed in the guide section (94, 132) has, at its lower end, a pin catch (96) for receiving the radial guide pin (49, 136) to define a direction of rotation of the actuation device (90, 162).

7. Dispenser according to any of the preceding Claims, **characterized in that**
for the purpose of defining an initial position of the actuation device (24, 162), in which the pumping devices (34, 35) are not acted upon by the transmission element (40, 146), a spring device is arranged between a stop (59, 147) formed at the guide section (44, 132) and the transmission element (40, 146).

8. Dispenser according to Claim 7,
**characterized in that**
the stop (59, 147) is formed at the axial end of a pin (58, 144) formed at the bottom of the guide section (44, 132) and extending through an opening formed in a bottom (60, 143) of a central cup-shaped indentation of the transmission element (40, 146), and **in that** the spring device is formed as a helical spring (61, 148) which is arranged in an annular space formed between the pin and the indentation and which extends between the stop and the bottom.

9. Dispenser according to any of Claims 1 to 6,
**characterized in that**.
for the purpose of defining an initial position of the actuation device (72), in which the pumping devices are not acted upon by the transmission element (75), a latching device (80) is arranged at an axial end of a latching pin (78), which is arranged in the guide section (76), said latching device, in the transmission element (75) being disposed in the initial position, engages behind an edge of a central opening (82) in the transmission element (75).

10. Dispenser according to any of the preceding Claims,
**characterized in that**
the guide path arranged in the inner wall (83) of the actuating housing (74) is formed as a guide groove (85), the groove edges (86, 87) thereof, which run in parallel to one another, receiving the guide pin (49) between them.

11. Dispenser according to any of the preceding Claims,
**characterized in that**
for forming the second guide device, the guide section (44, 76, 94) is formed by the adjusting housing (43, 73, 92) and the pin arrangement with the at least one guide pin (49) is formed at the transmission element (40, 75, 114), such that when the adjusting housing is rotated, the transmission element is correspondingly rotated as well.

12. Dispenser according to any of the preceding Claims,
**characterized in that**
for the purpose of the defined relative arrangement of the adjusting housing (43, 73, 92) with respect to the reservoir housing (21), a latching device (41, 97, 111) is formed between the adjusting housing (43, 73, 92) and the reservoir housing (21), which latching device has a plurality of latching positions for a defined assignment of contact regions of the transmission element (40, 75, 114) being formed on the contact surface (45) and plungers (38, 39) acting on pumping pistons (36, 37) of the pumping devices (34, 35).

13. Dispenser according to Claim 12,
**characterized in that**
the latching device (97) is formed as a modular latching unit which can be inserted between the reservoir housing (21) and the adjusting housing (92) and which can be actuated as a function of the direction of rotation with respect to the reservoir housing to enable the relative rotation of the adjusting housing.

14. Dispenser according to Claim 13,
**characterized in that**
the latching unit has a first latching element (98) of an annular design which can be connected to the reservoir housing (21) in a rotationally fixed manner and a second latching element (99) of an annular design which can be connected to the adjusting housing (92) in a rotationally fixed manner, wherein the latching elements interact with one another via a latching engagement which is produced in a common annular plane with the aid of latching projections, said latching projections being formed by a toothed pawl section (106, 107).

15. Dispenser according to Claim 14,
**characterized in that**
one of the two latching elements (98, 99) has the toothed pawl section (106, 107) only in a ring segment.

16. Dispenser according to Claim 12,
**characterized in that**
the latching device (11) has a latching axle (112) which is connected to the reservoir housing (21) and which engages with a hub of the transmission element (114) being formed as a latching sleeve (113) to produce a latching engagement.

17. Dispenser according to Claim 16,
**characterized in that**
latching projections formed on the circumference of the latching axle (112) interact with latching projections formed on the bore wall of the latching sleeve (113) to produce the latching engagement.

18. Dispenser according to Claim 17,
**characterized in that**
the latching projections are formed by a toothed pawl section (124).

19. Dispenser according to any of Claims 1 to 10,
**characterized in that**
for forming the second guide device, the guide section (132) is connected to the reservoir housing (21) in a rotationally fixed manner, the pin arrangement being formed as a guide ring (137) having at least one guide pin (136) and the transmission element (146) being received in the guide ring so as to be rotatable and being connected to the adjusting housing (131) in a rotationally fixed manner by means of a radial engagement device, such that when the adjusting housing is rotated, a relative rotation with respect to the guide section is effected by the same as well.

20. Dispenser according to Claim 19,
**characterized in that**
the engagement device has a setting sleeve (151) in which the transmission element (146) is received in a rotationally fixed manner and so as to be axially displaceable, wherein the setting sleeve produces an engaging connection to the adjusting housing (131) via a setting pin (152) which penetrates radially through the guide section in a setting groove (153).

21. Dispenser according to Claim 20,
**characterized in that**
for the purpose of the defined relative arrangement of the adjusting housing (131), a latching device (171) is formed between the setting sleeve (151) and the guide section (132), which latching device has a plurality of latching positions for a defined assignment of contact regions of the transmission element (146) being formed on the contact surface (159) and plungers (38, 39) acting on pumping pistons (36, 37) of the pumping devices (34, 35).

22. Dispenser according to any of the preceding Claims,
**characterized in that**
the contact regions are formed on the contact surface (45, 159) of the transmission element (40, 75, 146) by contact ledges (46, 170) which are arranged in a stepped sequence.

23. Dispenser according to Claim 22,
**characterized in that**
the contact ledges (46) are formed by blind bores (48) which are arranged in a horizontal surface of the transmission element (40, 75).

## Revendications

1. Distributeur doseur (20, 88, 110) pour la distribution dosée d'au moins deux composants, chacun étant reçu dans un réservoir (26, 27), un dispositif de pompage (34, 35) étant attribué à chaque réservoir (26, 27) et les dispositifs de pompage étant pourvus d'un dispositif d'actionnement (24, 72, 90, 162) pour distribuer les composants à partir d'un dispositif de distribution (31) relié aux réservoirs (26, 27), le dispositif d'actionnement agissant indirectement sur les dispositifs de pompage par l'intermédiaire d'un dispositif de dosage et le dispositif de dosage étant pourvu d'un dispositif de réglage (23, 71, 89, 161), la position d'un élément de transmission (40, 75, 114, 146), qui agit sur les dispositifs de pompage, pouvant être changée par l'actionnement du dispositif de réglage afin de régler le rapport quantitatif des composants,
**caractérisé en ce que**
tant le dispositif de réglage (23, 71, 89, 161) pour régler le rapport quantitatif des composants que le dispositif d'actionnement (24, 72, 90, 162) pour distribuer les composants à partir du dispositif de distribution (31) peuvent être tournés autour d'un axe longitudinal (25) du distributeur doseur (20, 88, 110) afin d'être actionnés.

2. Distributeur doseur selon la revendication 1,
**caractérisé en ce que**
le dispositif de réglage (23, 71, 89, 161) du dispositif de dosage a un boîtier de réglage (43, 73, 92, 131) qui peut être tourné autour de l'axe longitudinal (25) d'un boîtier de réservoir (21), dans lequel les réservoirs (26, 27) pour recevoir les composants et les dispositifs de pompage (34, 35) sont logés, l'élément de transmission (40, 75, 114, 146) étant disposé dans le boîtier de réglage de manière solidaire en rotation par rapport au boîtier de réglage et axialement déplaçable sur l'axe longitudinal du boîtier de réglage pour l'actionnement du dispositif de pompage, l'élément de transmission ayant une surface de contact (45, 139) en forme annulaire ou formé comme segment d'anneau (160), la surface de contact ayant un contour superficiel qui change dans le sens de la circonférence et interagit avec des pistons de pompage (36, 37) des dispositifs de pompage.

3. Distributeur doseur selon la revendication 2,
**caractérisé en ce que**
le dispositif d'actionnement (24, 72, 90, 162) pour la distribution des composants a un boîtier d'actionnement (54, 74, 93, 139) qui peut être tourné autour de l'axe longitudinal (25) du distributeur doseur (20) par rapport au boîtier de réglage (43, 73, 92, 131) et qui est pourvu d'un agencement de guidage pour permettre le déplacement axiale de l'élément de transmission (40, 75, 114, 146), un premier dispositif de guidage de l'agencement de guidage, qui est formé dans le boîtier d'actionnement, interagissant avec un deuxième dispositif de guidage de l'agencement de guidage formé indépendamment du boîtier d'actionnement afin de convertir un mouvement rotatif du boîtier d'actionnement en un mouvement axiale de l'élément de transmission.

4. Distributeur selon la revendication 3,
**caractérisé en ce que**
le deuxième dispositif de guidage comprend une partie de guidage (44, 76, 94, 132) ayant une coulisse de guidage (50, 95, 135) axialement orientée et un agencement de broches positionnée sur l'élément de transmission (40, 75, 114, 146) et ayant au moins une broche de guidage (49, 136) radiale qui pénètre la coulisse de guidage et, avec son extrémité de guidage, interagit avec le premier dispositif de guidage du boîtier d'actionnement (54, 74, 93, 139).

5. Distributeur doseur selon la revendication 3 ou 4,
**caractérisé en ce que**
pour la formation du premier dispositif de guidage, sur sa paroi intérieure (51, 83, 140) disposée coaxialement à la partie de guidage (44, 76, 94, 132), le boîtier d'actionnement (54, 74, 93, 139) a une voie de guidage qui a un contour de contact (64, 142) interagissant avec l'extrémité de contact de la broche de guidage (49, 136) et commandant le mouvement axial de l'élément de transmission (40, 75, 114, 146).

6. Distributeur doseur selon la revendication 4 ou 5,
**caractérisé en ce qu'**
à son extrémité inférieure, la coulisse de guidage (95, 135) formée dans la partie de guidage (94, 132) et axialement orientée a une piège de broche (96) destinée à recevoir la broche de guidage (49, 136) radiale afin de définir un sens de rotation du dispositif d'actionnement (90, 162).

7. Distributeur doseur selon l'une quelconque des revendications précédentes,
**caractérisé en ce que**
pour la définition d'une position de départ du dispositif d'actionnement (24, 162), dans laquelle l'élément de transmission (40, 146) n'agit pas sur les dispositifs de pompage (34, 35), un dispositif de ressort est disposé entre une butée (59, 147) disposée sur la partie de guidage (44, 132) et l'élément de transmission (40, 146).

8. Distributeur doseur selon la revendication 7,
**caractérisé en ce que**
la butée (59, 147) est formée à l'extrémité axiale d'une broche (58, 144) disposée sur le fond de la partie de guidage (44, 132), ladite broche s'étendant à travers une ouverture formée dans un fond (60, 143) d'un renfoncement central en forme de coupe de l'élément de transmission (40, 146), et que le dispositif de ressort est formé comme un ressort hélicoïdal (61, 148) qui est disposé dans un espace annulaire formé entre la broche et l'enfoncement et qui s'étend entre la butée et le fond.

9. Distributeur doseur selon l'une quelconque des revendications 1 à 6,
**caractérisé en ce que**
pour la définition d'une position de départ du dispositif d'actionnement (72), dans laquelle l'élément de transmission (75) n'agit pas sur les dispositifs de pompage, un dispositif d'encliquetage (80) est disposé à une extrémité axiale d'une broche d'encliquetage (78) disposée dans la partie de guidage (76), ledit dispositif d'encliquetage s'accrochant à un bord d'une ouverture centrale (82) dans l'élément de transmission (75) quand l'élément de transmission (75) se trouve dans sa position de départ.

10. Distributeur doseur selon l'une quelconque des revendications précédentes,
**caractérisé en ce que**
la voie de guidage disposée dans la paroi intérieure (83) du boîtier d'actionnement (74) est formée comme rainure de guidage (85) dont les bords de rainure (86, 87) s'étendant parallèlement l'un par rapport à l'autre reçoivent la broche de guidage (49) entre eux.

11. Distributeur doseur selon l'une quelconque des revendications précédentes,
**caractérisé en ce que**
pour la formation du deuxième dispositif de guidage, la partie de guidage (44, 76, 94) est formée par le boîtier de réglage (43, 73, 92), et l'agencement de broches est formé avec l'au moins une broche de guidage (49) sur l'élément de transmission (40, 75, 114) de telle manière que lors d'une rotation du boîtier de réglage, une rotation correspondante de l'élément de transmission s'effectue.

12. Distributeur doseur selon l'une quelconque des revendications précédentes,
**caractérisé en ce que**
pour la disposition relative définie du boîtier de réglage (43, 73, 92) par rapport au boîtier de réservoir (21), un dispositif d'encliquetage (41, 97, 111) est formé entre le boîtier de réglage (43, 73, 92) et le boîtier de réservoir (21), ledit dispositif d'encliquetage ayant une pluralité de positions d'encliquetage pour une attribution définie de points de contact de l'élément de transmission (40, 75, 114) formés sur la surface de contact (45) et de poussoirs (38, 39) agissant sur des pistons de pompage (36, 37) des dispositifs de pompage (34, 35).

13. Distributeur doseur selon la revendication 12,
**caractérisé en ce que**
le dispositif d'encliquetage (97) est formé comme unité d'encliquetage modulaire qui peut être insérée entre le boîtier de réservoir (21) et le boîtier de réglage (92) et qui peut être actionnée en fonction du sens de rotation afin de tourner relativement le boîtier de réglage par rapport au boîtier de réservoir.

14. Distributeur doseur selon la revendication 13,
**caractérisé en ce que**
l'unité d'encliquetage comprend un premier élément d'encliquetage (98) annulaire qui peut être relié de manière solidaire en rotation avec le boîtier de réservoir (21) et un deuxième élément d'encliquetage (99) annulaire qui peut être relié de manière solidaire en rotation avec le boîtier de réglage (92), les éléments d'encliquetage interagissant par un engagement par encliquetage formé dans un plan annulaire commun et produit par des projections d'encliquetage, et les projections d'encliquetage étant formées par une dentelure à cliquet d'arrêt (106, 107).

15. Distributeur doseur selon la revendication 14,
**caractérisé en ce qu'**
un des deux éléments d'encliquetage (98, 99) comporte la dentelure à cliquet d'arrêt (106, 107) dans seulement un segment d'anneau.

16. Distributeur doseur selon la revendication 12,
**caractérisé en ce que**
le dispositif d'encliquetage (111) a un essieu d'encliquetage (112) qui est relié avec le boîtier de réservoir (21) et qui engage dans un moyeu de l'élément de transmission (114) formé comme douille d'encliquetage (113) afin de former un engagement par encliquetage.

17. Distributeur doseur selon la revendication 16,
**caractérisé en ce que**
pour la formation de l'engagement par encliquetage, des projections d'encliquetage formées sur la circonférence de l'essieu d'encliquetage (112) interagissent avec des projections d'encliquetage formées sur la paroi d'alésage de la douille d'encliquetage (113).

18. Distributeur doseur selon la revendication 17,
**caractérisé en ce que**
les projections d'encliquetage sont formées par une dentelure à cliquet d'arrêt (124).

19. Distributeur doseur selon l'une quelconque des revendications 1 à 10,
**caractérisé en ce que**
pour la formation du deuxième dispositif de guidage, la partie de guidage (132) est reliée de manière solidaire en rotation avec le boîtier de réservoir (32), l'agencement de broches est formé comme anneau de guidage (137) ayant au moins une broche de guidage (136) et l'élément de transmission (146) est logé de manière rotative dans l'anneau de guidage et est relié de manière solidaire en rotation avec le boîtier de réglage (131) au moyen d'un dispositif d'engagement radial de telle manière que lors d'une rotation du boîtier de réglage, ce dernier effectue une rotation relative par rapport à la partie de guidage.

20. Distributeur doseur selon la revendication 19,
**caractérisé en ce que**
le dispositif d'engagement a une douille de réglage (151) dans laquelle l'élément de transmission (146) est logé de manière solidaire en rotation et axialement déplaçable, la douille de réglage formant une liaison par engagement avec le boîtier de réglage (131) par l'intermédiaire d'une broche de réglage (152) qui pénètre radialement la partie de guidage (132) dans une rainure de réglage (153).

21. Distributeur doseur selon la revendication 20,
**caractérisé en ce que**
pour la disposition relative définie du boîtier de réglage (131), un dispositif d'encliquetage (171) est formé entre la douille de réglage (151) et la partie de guidage (132), ledit dispositif d'encliquetage ayant une pluralité de positions d'encliquetage pour une attribution définie de points de contact de l'élément de transmission (146) formés sur la surface de contact (159) et de poussoirs (38, 39) agissant sur des pistons de pompage (36, 37) des dispositifs de pompage (34, 35).

22. Distributeur doseur selon l'une quelconque des revendications précédentes,
**caractérisé en ce que**
les points de contact sur la surface de contact (45, 159) de l'élément de transmission (40, 75, 146) sont formés par des épaulements de contact (46, 170) disposés en une séquence en gradins.

23. Distributeur doseur selon la revendication 22,
**caractérisé en ce que**
les épaulements de contact (46) sont formés par des alésages borgnes (48) qui sont disposés dans une surface horizontale de l'élément de transmission (40, 75).
